# EUROPEAN PATENT APPLICATION

(11) **EP 2 198 806 A2**
(43) Date of publication of application: **23.06.2010**
(21) Application number: 08805332.7
(22) Date of filing: 17.07.2008
(51) Int. Cl.: A61F 2/24

(54) **DEVICE FOR REPAIRING MITRAL VALVE INSUFFICIENCY**

(30) Priority: 17.07.2007 ES 200701987
(71) Applicant: Ilerimplant, S.L., Enginyer Mies, núm. 705 B (Pol.Ind. El Segre) 25191 Lleida (ES)
(72) Inventor: RUYRA BALIARDA, Francisco Javier, E-25191 Lleida (ES)
(74) Representative: Morgades y Manonelles, Juan Antonio
(86) International application number: PCT/ES2008/000503
(87) International publication number: WO 2009/010619

(57) **Abstract**

The device is associated with the conventional prosthetic ring, which is permanently attached to the natural ring of the mitral valve and consists of two pieces attached to each other in a sliding arrangement, which form a positioning mechanism. One of said pieces has an essentially T-shaped form for its temporary attachment to the conventional prosthetic ring during the surgical operation, and it has a handle which sticks out from the piece on the side facing out from the prosthetic ring; and the other piece is in the form of an arc with a curvature corresponding to the opening of the mitral valve, and possesses an orifice at the mid-point of the arc, into which the trunk of the T-shaped piece is inserted in order to slide the arc-shaped piece in relation to the T-shaped piece and fix it temporarily over one of the edges of the mitral valve in order to perform the repair of the same.

## Description

### OBJECT OF THE INVENTION

The object of this invention is a device to repair mitral valve insufficiency.

### FIELD OF THE INVENTION

The aim of this invention is to repair the mitral valve when the natural tendinous_cords stretch or break, such that the aforementioned valve does not close correctly during the heartbeat. In order to carry out the pumping of the heart correctly, and if the mitral valve does not form a perfect closure and at precisely the right moment, part of the blood pumped by the heartbeat flows into the left auricle later than it would normally.

When the mitral veils fail to close correctly due to this stretching or breaking of the natural cords, this is referred to as prolapse, that is, the "falling" of one of the two veils (front or rear) which perform the closing and opening of the mitral valve opening, or of both at the same time. In short, the deterioration of said natural tendinous cords varies the mobility of the mitral valve in closing and opening such that two alterations take place simultaneously: on the one hand, the mitral valve fails to close and open correctly and, on the other, neither of the two moments takes place correctly.

### BACKGROUND TO THE INVENTION

Up to now, when a mitral valve becomes seriously deteriorated, said valve has had to be replaced or repaired. Today, it has been established that repair is better than replacement. There are many techniques for the repair of the mitral valve, individualized for each different patient and each different pathology. In general, a prosthetic ring is always used (complete or incomplete, rigid, flexible or semi-rigid, or with different shapes), this being sown to the natural ring of the mitral valve in order for this to adopt the shape which is most suitable and closest to the original. Moreover, the veils (drying tissue), tendinous cords (shortening or replacing them), papillary muscles or ventricular wall are operated on.

The replacement of the natural tendinous cords for artificial goretex cords is a very useful and versatile tool in mitral valve repair. However, the implantation of these artificial cords has two aspects of complexity which have limited the application of the same and prevented many surgeons from being able to repair with guarantees. The first complex aspect is that of measuring the length of these artificial cords exactly such that they are neither too long nor too short, in which case the repair would be incorrect. The other aspect is being able to tie these gore-tex cords on to the veil without this manoeuvre causing said veil to slide downwards, thereby affecting the length measurement taken. This is the case, due to the slippery nature of the gore-tex suture. In order to attempt to avoid these problems, many techniques and manoeuvres have been described. None of these has been universally accepted and none shows clear or definitive advantages with regard to the rest. In summary, it is very difficult to make the artificial cords which replace the natural tendinous cords have the exact length to be able to perform the precise closure or opening of the mitral valve opening or orifice, and for the knots made in the artificial cords to remain in place and not slide.

### SUMMARY OF THE INVENTION

The aforementioned drawbacks have been totally eliminated by means of this invention which enables the artificial cords which replace the stretched or broken natural tendinous cords to have exactly the right length for the correct functioning of the mitral valve, and prevents the knots which hold said artificial cords in place from sliding and, therefore, the length of the artificial cords from varying.

In accordance with the foregoing, the device for repairing the insufficiency of the mitral valve, which is the object of this invention, is characterized essentially by being made up of two pieces which are joined in sliding form and which form a positioning mechanism. One of the pieces has an essentially T-shaped form for its temporary attachment to the conventional prosthetic ring during the surgical operation and possesses a handle which sticks out from the piece on the side facing out from the prosthetic ring; and the other piece is in the form of an arc with a curvature corresponding to the opening of the mitral valve and possesses an orifice at the mid-point of the arc into which the trunk of the T-shaped piece is inserted in order to slide the arc-shaped piece and fix it temporarily over the mitral valve in order to perform the repair of the insufficiency of the same.

According to the invention, the attachment of the T-shaped piece to the prosthetic ring is carried out by means of respective sutures of the trunk and branches of the T, at points near their ends, to the conventional prosthetic ring, the positioning of the arc-shaped piece to the front or rear veil of the essentially arc-shaped opening of the mitral valve being carried out by means of several sutures, at intervals, of the arc-shaped piece to the veil on one of the edges of the opening, and the handle being in the position corresponding to the split point and/or zone of the conventional prosthetic ring and can be lifted in order to raise the combination of the T-shaped and arc-shaped pieces, both free ends of the split point and/or zone of the prosthetic ring and at least one of the two veils, front or rear, of the mitral valve opening, with regard to the opposite zone of said prosthetic ring, which is raised to a height which makes it possible to carry out the operation of replacing the stretched or broken natural tendinous cords by artificial cords of a precise length, for the correct opening and closing of the same in the normal pumping of blood by the heart for the circulation of the same through the body.

The T-shaped piece and arc-shaped piece, which slide one through the other, and which form the positioning mechanism are made of stainless steel, the sutures for the attachment of the T-shaped piece to the conventional prosthetic ring and of the arc-shaped piece to the veil of the mitral valve being made of gore-tex material, and the artificial cords which replace the natural tendinous cords being made of gore-tex material whose knots are fixed and incapable of slipping.

These and other characterising features will be best made apparent by the following detailed description whose understanding will be made easier by the accompanying two sheets of drawings showing a practical embodiment being cited only by way of example not limiting the scope of the present invention.

### DESCRIPTION OF THE DRAWINGS

In the drawings:

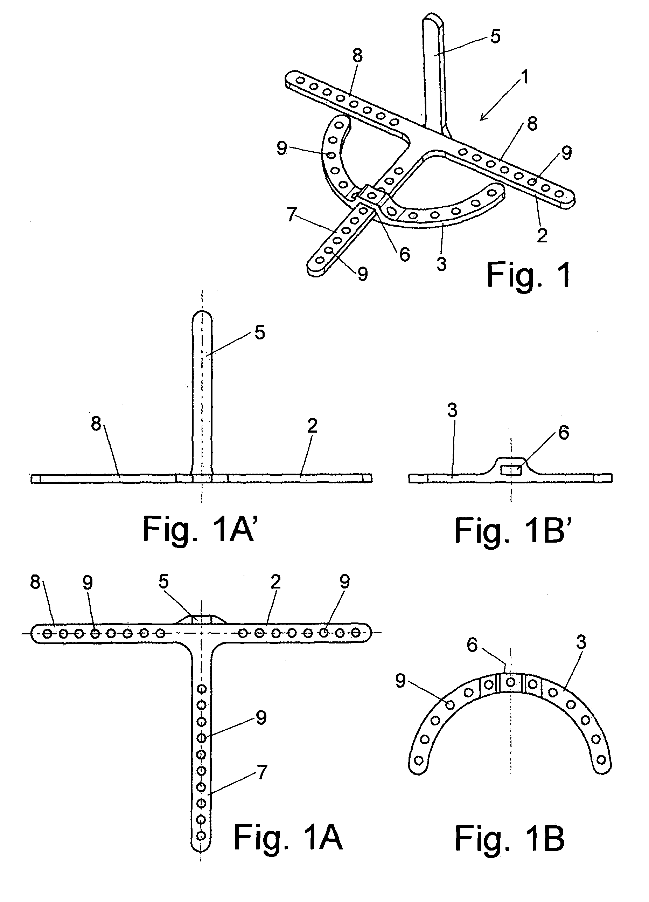

Figure 1 is a perspective view of device of the present invention.
Figure 1A is a plan view of the T-shaped piece of the device in Figure 1.
Figure 1A' is an elevation view T-shaped piece seen from the free and of the trunk of the T.
Figure 1 B is a plan view of the arc-shaped piece of the device in Figure 1.
Figure 1B' is an elevation view of the arc-shaped piece seen from the concave side.
Figure 2A is a plan view illustrating the conventional prosthetic ring split at a point and permanently fixed to the mitral valve.
Figure 2B is a similar view to Figure 2A, in which the prosthetic ring is split in an area instead of at a point.
Figure 3 is a plan view illustrating the prosthetic ring split at a point, as in Figure 2A, with the invention device temporarily attached to said prosthetic ring, in order to perform the corresponding surgical operation.
Figure 4 is a perspective view showing a schematic illustration of the mitral valve to be operated and the prosthetic ring of the type split at a point in a raised position by said point for the performance of the operation.
Figure 5 is a perspective view similar to Figure 4 but with the invention device temporarily attached to the prosthetic ring, which, by acting on the handle, causes the raising of the combination of pieces illustrated in Figure 4.
Figure 6 is a view which shows, schematically, the replacement of the stretched or broken natural tendinous cords in the performance of the mitral valve repair operation.

### DETAILED DESCRIPTION OF ONE FORM OF PREFERRED EMBODIMENT OF THE INVENTION

According to the drawings, the device of the invention with general reference -1- is formed by two pieces -2-, -3- joined together in sliding form, which form a positioning mechanism. Piece -2- has essentially T-shaped form for its temporary attachment to the conventional prosthetic ring -4- during the surgical operation and possesses a handle which sticks out from the piece on the side facing out from the prosthetic ring -4- (see figures), said prosthetic ring, in turn, being previously and permanently sown to the natural ring of the mitral valve.

And the other piece -3- is in the form of an arc with a curvature corresponding to the opening or orifice -A- of the mitral valve -MV- and possesses an orifice - 6- at the mid-point of the arc (see figures, especially Figure 1) into which the trunk -7- of the T-shaped piece is inserted in order to slide the arc-shaped piece -3- with regard to the T-shaped piece -2- and fix it temporarily over the mitral valve -MV- in correspondence with the opening -A- in order to perform the repair of the same.

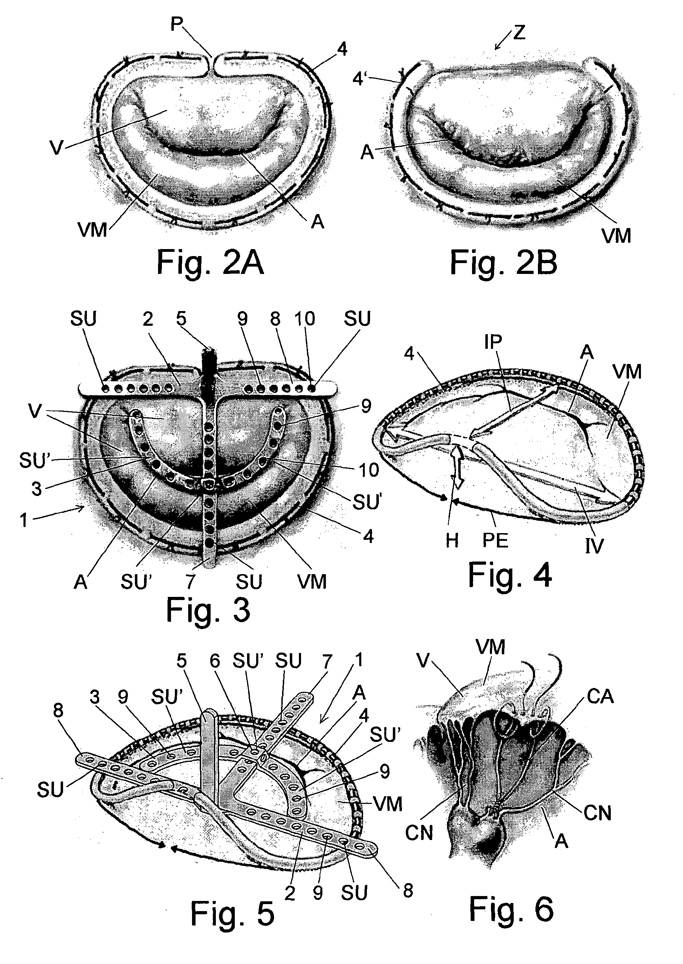

In order to perform the mitral valve -VM- operation with the device -1- of this invention, the T-shaped piece -2- is attached to the prosthetic ring -4-, see Figure 3, by means of respective sutures -SU- of the trunk -7- and branches -8-8- of the T-shaped piece, at points close to their ends, to the conventional prosthetic ring -4-, for which purpose said trunk -7- and branches -8-8- of the T-shaped piece -2- possess respective series of orifices -9- to attach said T-shaped piece to the prosthetic ring -4- by means of filaments -10-, preferably of GORE-TEX (registered trademark) material whose knots do not slip. Likewise, the arc-shaped piece -3- is positioned in relation to the front or rear veil -V- of the essentially arc-shaped opening or orifice -O- of the mitral valve -MV- by means of several sutures -SU- spaced between a few orifices of a sequence of orifices -9- provided in the arc-shaped piece -3- and one of the edges of the opening -0- of the veil -V-. To carry out the surgical operation on the mitral valve -MV-, the position of the device -1- of the invention is that illustrated in figures 3 and 5.

As illustrated, the handle -5- is in the position corresponding either to the point -P- at which the prosthetic ring is split, as illustrated in the aforementioned figures 3, 5 and 2A and 4, or to the zone -Z- in which the conventional prosthetic ring -4- is split or interrupted (incomplete), the handle of which can be raised in order to lift the combination of the T-shaped piece -2- and arc-shaped piece -3-, both free ends of the split point or zone of the prosthetic ring - 4- and at least one of the front or rear veils -V- of the opening -0- of the mitral valve -MV-, in relation to the opposing zone of said prosthetic ring, as illustrated in figure 4. Said combination of pieces is raised to a height -H- which makes it possible to carry out the operation -which is illustrated schematically, half way through, in figure 6 -- of replacing the stretched or broken natural tendinous cords -CN- already replaced in said figure 6 by artificial cords -AC- of precise length, for the correct opening and closing of the opening or orifice -O- of the aforementioned mitral valve -MV- in the normal pumping of the blood by the heart for the circulation of the same through the body, in the combination of the auricular and mitral valves of each side of the heart.

In Figure 4 the arrows indicate the aforementioned height -H-, the inter-peak distance -IP-, the intertrough distance -IT- and the perimeter -PE.

In the device -1- of the of the invention, made up of the T-shaped piece -2- and the arc-shaped piece -3-, which slide through one another, and form the positioning mechanism, both pieces are made of stainless steel. The arc-shaped piece -3- has a curvature corresponding to that of the opening -0- of the mitral valve -MV- in question, for which purpose the surgical team has several different arc-shaped pieces -3- for different curvatures of the mitral valve openings, in order to use the arc-shaped piece -3- which corresponds with the curvature of the opening -O- of the mitral valve of the patient to be operated. Meanwhile, the sutures -SU- for the attachment of the T-shaped -2- to the conventional prosthetic ring -4- and the sutures - SU- of the arc-shaped piece -3- to the veil -V- of the mitral valve -MV- are filaments of gore-tex material. Likewise, the artificial cords -AC- which replace the natural tendinous cords -NC- are cords of gore-tex material whose knots are firm and incapable of slipping.

## Claims

1. Device for e type repairing mitral valve insufficiency, of the type associated with the conventional prosthetic ring (4) which is split at a point (P) and/or zone (Z) of the same and which is permanently attached to the natural ring of the mitral valve (MV) in order for it to adopt the most suitable natural shape, **characterized by** consisting of two pieces (2,3), attached to each other in a sliding arrangement, which form a positioning mechanism. One of said pieces (2) has an essentially T-shaped form for its temporary attachment to the conventional prosthetic ring (4) during the surgical operation, and it has a handle (5) which sticks out from the piece on the side facing out from the prosthetic ring (4); and the other piece (3) is in the form of an arc with a curvature corresponding to the opening (O) of the mitral valve (MV) and possesses an orifice (6) at the mid-point of the arc, into which the trunk (7) of the T-shaped piece is inserted in order to slide the arc-shaped piece (3) in relation to the T-shaped piece (2) and fix it temporarily over the mitral valve (MV) in order to perform the repair of the same.

2. Device, according to claim 1, **characterized by** the fact that the attachment of the T-shaped piece (2) takes place by means of respective sutures (SU) of the trunk (7) and branches (9) of the T-shaped piece (2), at positions near the ends of the same, to the conventional prosthetic ring (4), the positioning of the arc-shaped piece (3) to the front or rear veil (V) of the essentially arc-shaped opening (O) of the mitral valve (MV) being carried out by means of several sutures (SU), at intervals, of the arc-shaped piece (3) to the veil (V) on one of the edges of the opening (O), and the handle (5) being in the position corresponding to the split point (P) and/or zone (Z) of the conventional prosthetic ring (4) and can be lifted in order to raise the combination of the T-shaped (2) and arc-shaped (3) pieces, both free ends of the split point (P) and/or zone (Z) of the prosthetic ring and at least one of the two veils (V), front or rear, of the opening (O) of the mitral valve (MV), in relation to the opposite zone of said prosthetic ring (4), which is raised to a height (H) which makes it possible to carry out the operation of replacing the stretched or broken natural tendinous cords (NC) by artificial cords (AC) of a precise length, for the correct opening and closing of the same in the normal pumping of blood by the heart for the circulation of the same through the body.

3. Device, according to claims 1 and 2, **characterized by** the fact that the T-shaped piece (2) and arc-shaped piece (3), which slide through one another, and which form the positioning mechanism are made of stainless steel, the sutures (SU) for the attachment of the T-shaped piece (2) to the conventional prosthetic ring (4) and of the arc-shaped piece (3) to the veil (V) of the mitral valve (MV) being made of gore-tex material, and the artificial cords (AC) which replace the natural tendinous cords (NC) being made of gore-tex material whose knots are fixed and incapable of slipping.
